# EUROPEAN PATENT APPLICATION

(11) **EP 2 386 278 A1**
(43) Date of publication of application: **16.11.2011**
(21) Application number: 10729227.8
(22) Date of filing: 07.01.2010
(51) Int. Cl.: A61F 13/15, A61F 13/472, A61F 13/511, A61F 13/56

(54) **ABSORBENT ARTICLE**

(30) Priority: 07.01.2009 JP 2009002086
(71) Applicant: Unicharm Corporation, Ehime 799-0111 (JP)
(72) Inventor: KUDO, Jun, Kanonji-shi Kagawa 769-1602 (JP); KINOSHITA, Hideyuki, Kanonji-shi Kagawa 769-1602 (JP); TAKAHASHI, Yuji, Kanonji-shi Kagawa 769-1602 (JP); MINAMI, Mari, Kanonji-shi Kagawa 769-1602 (JP); PHICHETKITJAWAT, Sarinee, Kanonji-shi Kagawa 769-1602 (JP); HASHINO, Akira, Kanonji-shi Kagawa 769-1602 (JP)
(74) Representative: Peter, Julian
(86) International application number: PCT/JP2010/050105
(87) International publication number: WO 2010/079807

(57) **Abstract**

An absorbent article 1 according to the present invention includes a front wing unit 60A which is provided outside in a widthwise direction W of the absorbent article 1 more than an absorber 30, and is extensive to an outside in a widthwise direction W of the absorber 30 in an inside leg region X1 which corresponds to an inside leg portion of a wearer; and a gather 50 which is provided at each of edges 31 of the absorber 30, which is extensive along a longitudinal direction L of the absorbent article 1, and which prevents a bodily fluid of the wearer from leaking from the absorbent article 1. The front wing unit 60A has a base part 61 which is positioned at an innermost side in a widthwise direction W of the absorbent article 1 in the front wing unit 60A; and the leakage gather 50 is extensive to an outside in the widthwise direction W of the absorbent article 1 more than the base part 61 as seen in a plan view of the absorbent article 1.

## Description

### [Technical Field]

The present invention relates to an absorbent article which is provided with: a liquid permeable topsheet; a liquid impermeable backsheet; and an absorber which is provided between the topsheet and the backsheet.

### [Background Art]

Conventionally, an absorbent article such as a sanitary napkin or a panty liner is formed in a longitudinally elongated shape which is extensive from a front side (a belly side) to a rear side (a back side) of a wearer. In general, an absorbent article is provided with: a liquid permeable topsheet; a liquid impermeable backsheet; and an absorber which is provided between the topsheet and the backsheet.

A gather (a leakage preventing unit) for preventing a wearer's bodily fluid from leaking from the absorbent article is provided at such an absorbent article. The gather is provided along a longitudinal direction of the absorbent article, and has a fixed end which is to be fixed to each of the edges of the absorber.

Inside of the gather, in general, a cord-like member such as a rubber having elasticity is provided in an expanded state. Since the expanded cord-like member is likely to be restored in a state before expanded, the absorbent article rounds, whereby the cord-like member rises in an erection direction which is spaced from the topsheet with the fixed end being a start point. Thus, the absorbent article (in particular, a gather) easily comes into contact with a wearer's skin, making it possible to prevent a side leakage in which the bodily fluid leaks from each of the edges of the absorber (refer to Patent Document 1, for example).

Incidentally, since the absorbent article is required to prevent the absorbent article from being displaced from shorts, a front wing unit is provided. The front wing unit is folded to a crotch portion (an inside leg portion) of the shorts, and then, is stopped at the shorts. The front wing unit is provided outside in a widthwise direction of the absorbent article more than the absorber, and is extensive to the outside in a widthwise direction of the absorber.

### [Prior Art Document]

### [Patent Document]

### [Patent Document 1]

Japanese Unexamined Patent Application Publication No. 2000-288025

### [Summary of the Invention]

The conventional absorbent article described above has entailed the following problem. That is, there has been a case in which, if the gather falls down on the absorber, a bodily fluid passes through a top of the gather that has fallen down thereon, and a side leakage occurs. In order to restrain such a side leakage to its required minimum, it is considered to increase an area of the front wing unit, for example, to lengthen the front wing unit in a longitudinal direction of the absorbent article.

However, if the front wing unit is too long in the longitudinal direction of the absorbent article, it becomes difficult to fold the front wing unit back to the crotch portion of the shorts, and handling of the absorbent article becomes cumbersome. For example, if a wearer forcibly folds the front wing unit back to the crotch portion of the shorts, winkles occur at the topsheet or the front wing unit, and there is an apprehension that the wearer has a sense of discomfort while in use.

Therefore, it is an object of the present invention to provide an absorbent article which is capable of facilitating handling of the absorbent article without causing a wearer to have a sense of discomfort while is use and further reliably preventing a side leakage.

In order to solve the above described problem, the present invention has the following characteristics. First, a primary characteristic of the present invention is directed to an absorbent article (for example, an absorbent article 1) which is provided with: a liquid permeable topsheet (a topsheet 10); a liquid impermeable backsheet (a backsheet 20); and an absorber (an absorber 30) which is provided between the topsheet and the backsheet, said absorbent article being provided with: a front wing unit (a front wing unit 60A) which is provided outside in a widthwise direction of the absorbent article more than the absorber, and is extensive to the outside in a widthwise direction of the absorber in an inside leg region which corresponds to a wearer's inside leg portion; and a leakage preventing unit (for example, a gather 50) which is provided at each of the edges (each of the edges 31) of the absorber, and is extensive along a longitudinal direction of the absorber, for preventing the wearer's bodily liquid from leaking from the absorbent article, wherein: the front wing unit has a base part (a base part 61) which is positioned at the innermost side in the widthwise direction of the absorbent article in the front wing unit; and the leakage preventing unit is extensive to the outside in the widthwise direction of the absorber article more than the base part as seen in a plan view of the absorbent article.

According to the characteristic of the present invention, there can be provided an absorbent article which is capable of facilitating handling of the absorbent article without causing a wearer to have a sense of discomfort while is use and further reliably preventing a side leakage.

### [Brief Description of Drawings]

[Fig. 1] Fig. 1 is a plan view in a side part opened state of an absorbent article 1 according to a first embodiment.
[Fig. 2] Fig. 2 is a sectional view in the side part opened state of the absorbent article 1 according to the first embodiment.
[Fig. 3] Fig. 3 is a plan view in a side part closed state of the absorbent article 1 according to the first embodiment.
[Fig. 4] Fig. 4 is a sectional view in the side part closed state of the absorbent article 1 according to the first embodiment.
[Fig. 5] Fig. 5 is a plan view in a side part opened state of an absorbent article 1A according to a second embodiment.
[Fig. 6] Fig. 6 is a sectional view in the side part opened state of the absorbent article 1A according to the second embodiment.
[Fig. 7] Fig. 7 is a plan view in a side part closed state of the absorbent article 1A according to the second embodiment.
[Fig. 8] Fig. 8 is a sectional view in the side part closed state of the absorbent article 1A according to the second embodiment (a first view).
[Fig. 9] Fig. 9 is a sectional view in the side part closed state of the absorbent article 1A according to the second embodiment (a second view).
[Fig. 10] Fig. 10 is a plan view in a side part opened state of an absorbent article 1B according to a third embodiment.
[Fig. 11] Fig. 11 is a sectional view in the side part opened state of the absorbent article 1B according to the third embodiment (a first view).
[Fig. 12] Fig. 12 is a sectional view in the side part opened state of the absorbent article 1B according to the third embodiment (a second view).
[Fig. 13] Fig. 13 is a sectional view in a side part opened state of an absorbent article 1C according to Exemplary Modification 1 of the third embodiment.
[Fig. 14] Fig. 14 is a plan view in a side part opened state of an absorbent article 1D according to Exemplary Modification 2 of the third embodiment.
[Fig. 15] Fig. 15 is a plan view in a side part opened state of an absorbent article 1E according to Exemplary Modification 3 of the third embodiment.
[Fig. 16] Fig. 16 is a plan view in a side part opened state of a side part opened state of an absorbent article 1F according to a fourth embodiment.
[Fig. 17] Fig. 17 is a sectional view in the side part opened state of the absorbent article 1F according to the fourth embodiment.
[Fig. 18] Fig. 18 is a plan view in a side part opened state of an absorbent article 1G according to Exemplary Modification of the fourth embodiment.
[Fig. 19] Fig. 19 is a sectional view in the side part opened state of the absorbent article 1G according to Exemplary Modification of the fourth embodiment.

### [Description of Embodiments]

Hereinafter, an absorbent article according to the present invention will be described with reference to the drawings. Specifically, descriptions will be furnished with respect to a first embodiment, a second embodiment, a third embodiment, a fourth embodiment, and other embodiments.

In the description of the drawings that follows, the same or similar constituent elements are designated by the same or similar reference numerals. However, it should be kept in mind that: the drawings are merely schematic; and rates of dimensions or the like each are different from an actual one.

Therefore, specific dimensions or the like should be determined in consideration of the following description. It is a matter of course that portions whose dimensional interrelationships or rates are different from each other are included in the drawings as well.

### [First Embodiment]

### First, an absorbent article according to a first embodiment will be described with reference to the drawings.

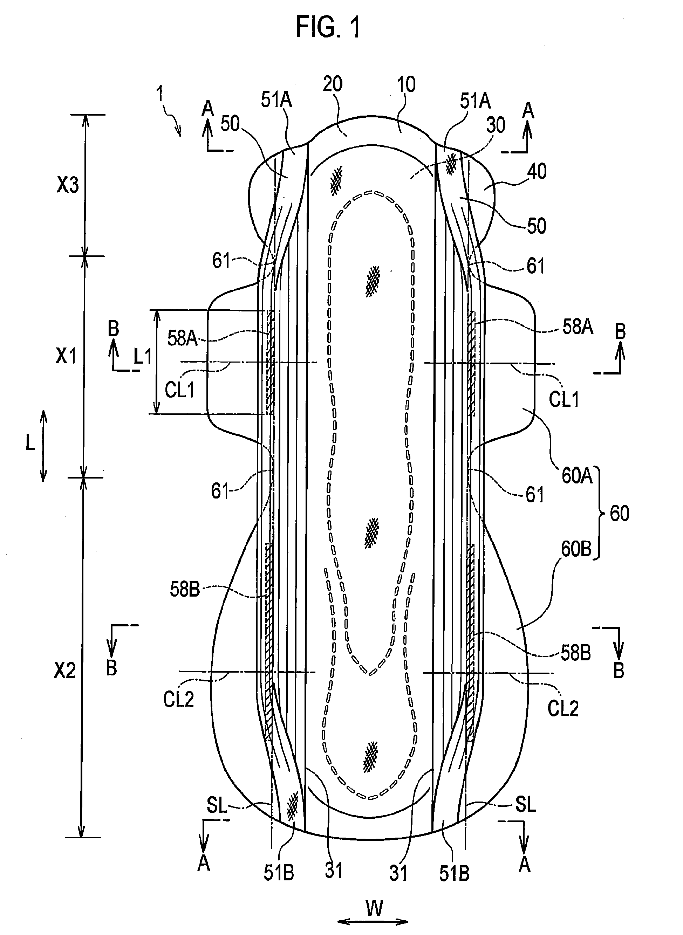

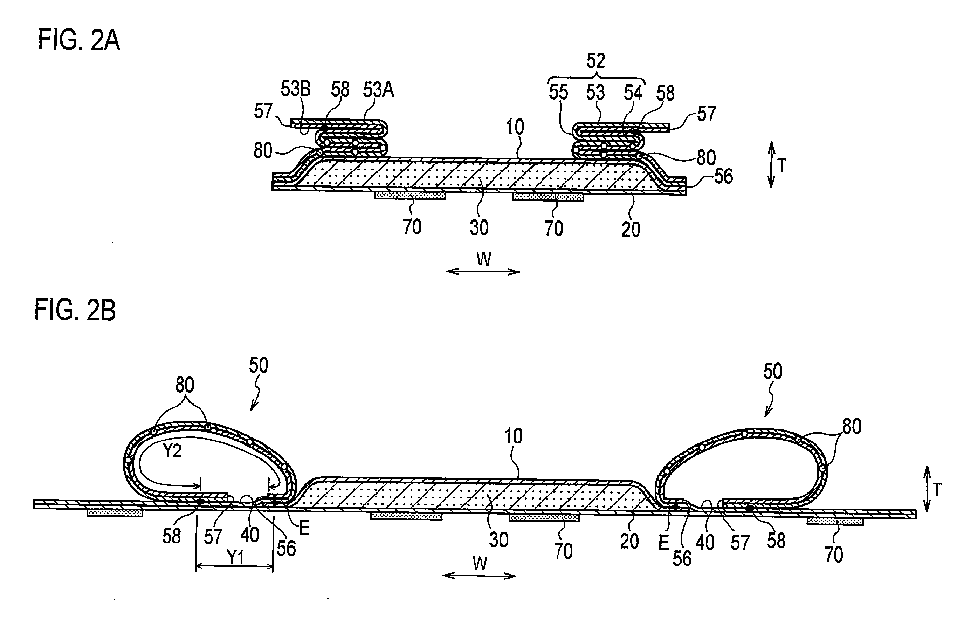

First of all, a structure of an absorbent article 1 according to the first embodiment will be described with reference to the drawings. Fig. 1 is a plan view in a side part opened state of the absorbent article 1 according to the first embodiment. Fig. 2 (a) is a sectional view in the side part opened state of the absorbent article 1 according to the first embodiment (the sectional view taken along the line A-A of Fig. 1). Fig. 2 (b) is a sectional view in the side part opened state of the absorbent article 1 according to the first embodiment (the sectional view taken along the line B-B of Fig. 1).

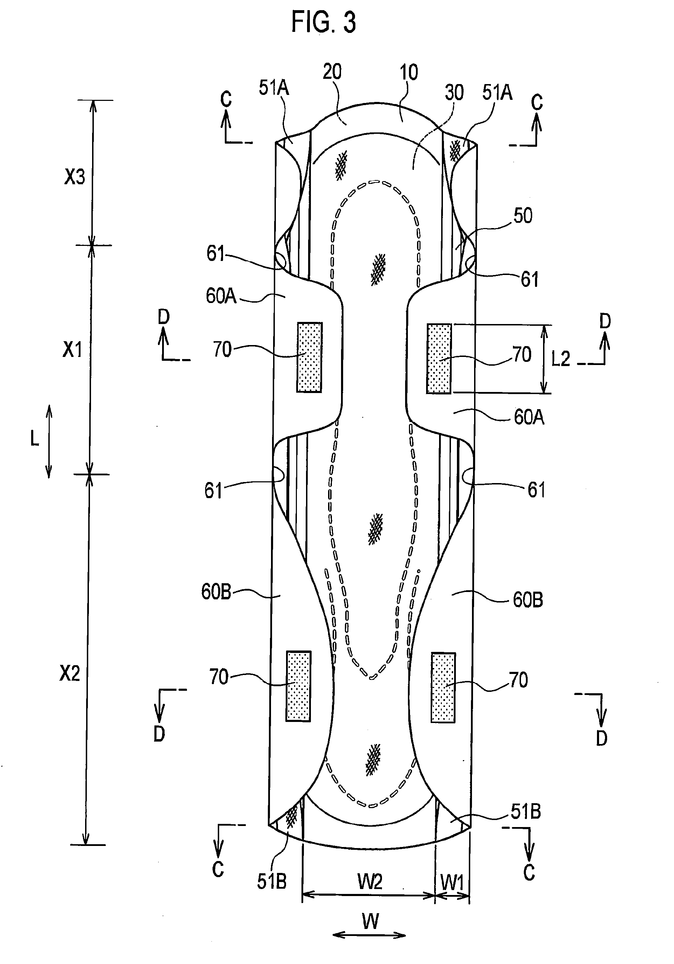

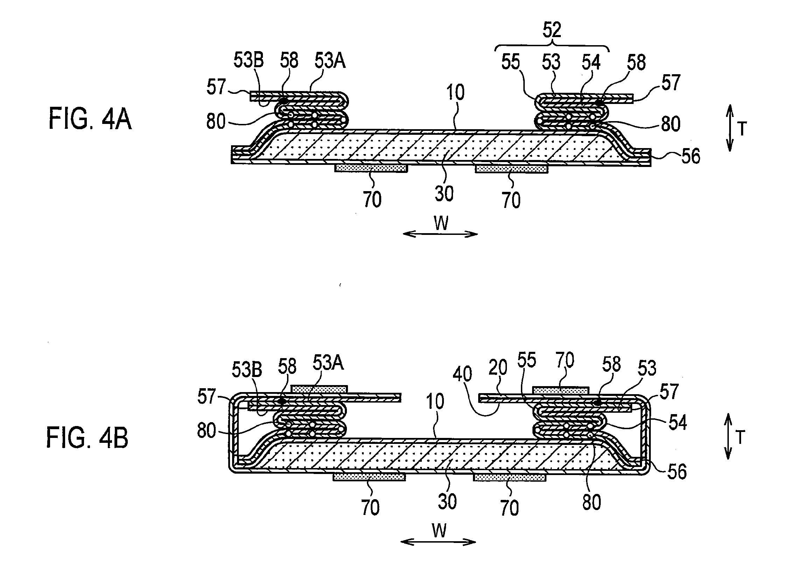

Fig. 3 is a plan view in a side part closed state of the absorbent article 1 according to the first embodiment. Fig. 4 (a) is a sectional view in the side part closed state of the absorbent article 1 according to the first embodiment (the sectional view taken along the line C-C of Fig. 3). Fig. 4 (b) is a sectional view in the side part closed state of the absorbent article 1 according to the first embodiment (the sectional view taken along the line D-D of Fig. 3). In the first embodiment, the absorbent article 1 is employed as a sanitary napkin.

As shown in Fig. 1 to Fig. 4, the absorbent article 1 is formed in a longitudinally elongated shape which is extensive from a front side to a rear side of a wearer. The absorbent article 1 is provided with: a liquid permeable topsheet 10 which comes into contact with a skin of the wearer; a liquid impermeable backsheet 20 which is provided at a non-wearer's side more than the topsheet 10; an absorber 30 which is provided between the topsheet 10 and the backsheet 20; and a side sheet 40 which is provided outside in a widthwise direction W in a widthwise direction of the absorber 30 which is orthogonal to a longitudinal direction L of the absorbent article 1.

In addition, the absorbent article 1 is further provided with: a gather 50 (a leakage preventing unit) which is provided at each of the edges 31 of the absorber 30, and is extensive along the longitudinal direction L of the absorbent article 1; and a wing unit 60 which is provided outside in the widthwise direction W of the absorbent article 1 more than the absorber 30, and is extensive outside in the widthwise direction W of the absorber 30. A structure of the gather 50 will be described later.

The wing unit 60 is comprised of a backsheet 20 and a side sheet 40. The wing unit 60 is different from the gather 50 in hue. The wing unit 60 is formed of a liquid impermeable material. It is preferable that the wing unit 60 is 150 mm or less (in particular, 100 mm or less) in bending rigidity employing a cantilever.

The wing unit 60 is comprised of a front wing unit 60A which is folded back to a crotch portion (an inside leg portion) of shorts and then is stopped at the shorts; and a rear wing unit 60B which is provided at a rear side of a wearer more than the front wing unit 60A, and is extensive to the outside in the widthwise direction W of the absorbent article 1 along the shape of the shorts.

The front wing unit 60A is extensive to the outside in the widthwise direction W of the absorber 30 in an inside leg region X1 which corresponds to an inside leg portion of the wearer. At the front wing unit 60A, a base part 61 which is positioned at the innermost side in the widthwise direction W of the absorbent article 1 in the front wing unit 60A is formed.

The rear wing unit 60B is extensive to the outside in the widthwise direction of the absorber 30 in a rear region X2 which corresponds to a rear side of the wearer more than the front wing unit 60A. Namely, the rear wing unit 60B is provided at the rear side of the wearer more than the front wing unit 60A.

A displacement proof unit 70 (for example, a hot melt adhesive), which has an adhesive property of preventing the absorbent article 1 from being displaced from shorts, is provided at the wing unit 60 (the front wing unit 60A and the rear wing unit 60B) or on a face at the side of the backsheet 20 in the absorber 30.

Next, a structure of the abovementioned gather 50 will be described with reference to Fig. 1 to Fig. 4.

As shown in Fig. 1 to Fig. 4, the gather 50 is adapted to prevent the wearer's bodily fluid from leaking from the absorbent article 1. The gather 50 is formed of an nonwoven cloth or a perforated film.

The gather 50 is formed of a sheet which is different from the topsheet 10 or the side sheet 40. The gather 50 does not always need to be comprised of another sheet which is different from the topsheet 10 or the side sheet 40, and the gather may be formed by means of extension of the side sheet 40.

As shown in Fig. 1 and Fig. 2, the gather 50 rises in a direction spaced from the topsheet 10 (hereinafter, referred to as an erection direction T), i.e., toward the wearer's side, at the time of wearing it. In addition, as shown in Fig. 3 and Fig. 4, the gather 50 is folded back onto the topsheet 10 a plurality of times in a side part closed state of the absorbent article 1, i.e., in a state in which the gather 50 does not rise.

Here, the side part closed state of the absorbent article 1 is a state in which, when the wing unit 60 is folded back to the side of the topsheet 10 from a boundary between the absorber 30 and the wing unit 60, the gather 50 is disposed on the topsheet 10, together with the wing unit 60, and the gather 50 is folded on the topsheet 10 a plurality of times. In the side part closed state of the absorbent article 1, a mold release sheet (not shown) which covers the displacement proof unit 70 may be provided between the displacement proof unit 70 that is disposed at the uppermost side and a packing member (not shown).

Inside of the gather 50, a cord-like member 80 (refer to Fig. 2 and Fig. 4) such as a rubber, which is disposed along the longitudinal direction L of the absorbent article 1 and has elasticity, is provided in an expanded state. The gather 50 is provided with a front end portion 51A which is positioned at the front side of the wearer and a rear end portion 51B which is positioned at the rear side of the wearer.

At the front end portion 51A and the rear end portion 51B, a hot melt adhesive is applied between layers of the gather 50 that is folded on the topsheet 10. In addition, at the front end portion 51A and the rear end portion 51B, emboss treatment is applied to all of the layers of the gather 50 that is folded on the topsheet 10 (not shown). In this manner, at the front end portion 51A and the rear end portion 51B, the gather 50 is folded a plurality of times, and is bonded with the absorbent article 1 in a state in which the gather is folded on the topsheet 10.

In this manner, the gather 50 rises toward the wearer's side at the time of wearing it, and a hollow part is formed in each of an inside leg region X1 and a rear region X2. Such a gather 50 has a gather main unit 52, a fixed end 56, and a free end 57.

The gather main unit 52 is formed in a sheet shape along the longitudinal direction L of the absorbent article 1. The gather main unit 52 has: at least an outermost piece portion 53 which is positioned at the most wearer's side in a state in which the gather 50 is folded on the topsheet 10 a plurality of times as seen in a sectional view (refer to Fig. 4) in the widthwise direction W of the absorbent article 1; a lower piece portion 54 which is positioned at the side of each of the edges 31 of the outermost piece portion 53; and a continuous portion 55 which connects the outermost piece portion 53 and the lower piece portion 54 to each other.

The outermost piece portion 53 has: an outermost face 53A which is positioned at the most wearer's side in the erection direction T of the gather 50; and an innermost face 53B which is positioned at an opposite side of the outermost face 53A, i.e., at the side of the lower piece portion 54.

A fixed end 56 is provided along the longitudinal direction L of the absorbent article 1, and is fixed (bonded) by an emboss part E being applied to each of the edges 31. The fixed end 56 may be provided at each of the edges 31 of the absorber 30, may be provided outside in the widthwise direction W more than the absorber 30, or may be provided on each of the edges of the absorber 30.

The fixed end 56 serves as a start point when the gather 50 rises in the erection direction T. Namely, the gather 50 rises in the erection direction T spaced from the topsheet 10 with the fixed end 56 being a start point.

The free end 57 is provided along the longitudinal direction L of the absorbent article 1, and is positioned at the wearer's side more than each of the edges 31. The free end 57 is oriented to the outside in the widthwise direction W of the absorbent article 1 in a state in which the gather 50 is folded on the topsheet 10 a plurality of times. Specifically, the free end 57 is positioned at a tip end part of the outermost piece portion 53, and is provided outside in the widthwise direction W of the absorbent article 1 more than the continuous portion 55. The free end 57 is provided between the gather main unit 52 and the wing unit 60 (the side sheet 40) (refer to Fig. 2 and Fig. 4).

Such a gather 50 is extensive to the outside in the widthwise direction W of the absorbent article 1 more than the base part 61 as seen in a plan view of the absorbent article 1 (refer to Fig. 1 and Fig. 3). Namely, the gather 50 is superimposed on the base part 61. In addition, at least a part of the gather 50 is bonded with the wing unit 60 at a bonding portion 58 by means of an adhesive agent such as a hot melt adhesive.

Next, a disposition site of the abovementioned bonding portion 58 will be described with reference to Fig. 1 to Fig. 4.

As shown in Fig. 1 to Fig. 4, at the bonding portion 58, the wing unit 60 and at least a part of the outermost piece portion 53 are bonded with each other. Specifically, in a state in which the gather 50 is folded on the topsheet 10 a plurality of times, the side sheet 40 and an outermost face 53A of the outermost piece portion 53 are bonded with each other at the bonding portion 58.

The bonding portion 58 is comprised of a front bonding portion 58A which is provided at a front wing unit 60A and a rear bonding portion 58B which is provided at a rear wing unit 60B.

The front bonding portion 58A is provided at a position which includes a centerline CL1 which passes through a center of the longitudinal direction L of the absorbent article 1 at the front wing unit 60A as seen in a plan view of the absorbent article 1, and is extensive along the widthwise direction W of the absorbent article 1. It is preferable that the front bonding portion 58A is provided to be uniform with respect to the centerline CL1.

A length L1 taken along the longitudinal direction L of the absorbent article 1 at the front bonding portion 58A (refer to Fig. 1) is larger than a length L2 taken along the longitudinal direction L of the absorbent article 1 at a placement proof unit 70 (refer to Fig. 3).

On the other hand, the rear bonding portion 58B is positioned at a position which includes a centerline CL2 which passes through the center of the longitudinal direction L of the absorbent article 1 at the rear wing unit 60B as seen in the plan view of the absorbent article 1, and is extensive along the widthwise direction W of the absorbent article 1.

The bonding portion 58 (the front bonding portion 58A and the rear bonding portion 58B) is positioned in the vicinity of a reference line SL which passes through the outermost side in the widthwise direction W of the base part 61, and which is extensive along the longitudinal direction L of the absorbent article 1. Specifically, the bonding portion 58 is positioned outside in the widthwise direction more than on the reference line SL. In particular, it is preferable that the bonding portion 58 is positioned between the reference line SL and a center part in the widthwise direction W of the wing unit 60.

Here, a length Y1 in the widthwise direction from an emboss part E (for example, an inside end which is positioned inside in the widthwise direction W of the emboss part E) to the bonding portion 58 (for example, an outside end which is positioned inside in the widthwise direction W of the bonding portion 58) is a length Y2 or less of the gather 50 from the emboss part E to the bonding portion 58 (Y1 ≤ Y2). Specifically, it is preferable that the length Y1 in the widthwise direction from the emboss part E to the bonding portion 58 is 1/2 or less, in particular 1/3 or less respect to the length Y2 of the gather 50 from the emboss part E to the bonding portion 58 (refer to Fig. 2).

The length Y2 of the gather 50 from the emboss part E to the bonding portion 58 is larger than a maximum length Y3 in the widthwise direction W of the gather 50 (Y2 ≥ Y3).

In the first embodiment, since the gather 50 is extensive to the outside in the widthwise direction W of the absorbent article 1 more than the base part 61 as seen in a plan view of the absorbent article 1, the gather 50 covers the base part 61. Thus, even if an area of the front wing unit 60A is increased, a side leakage can be reliably prevented. In this manner, it is possible to restrain the topsheet 10 or the front wing unit 60A from being wrinkled without the base part 61 being folded back to shorts, together with the wing unit 60. Therefore, the absorbent article 1 is easily handled without causing the wearer to have a sense of discomfort while in use.

Incidentally, if the gather 50 does not have the bonding portion 58, the gather 50 may fall down on the absorber 30 by the wearer's action at the time of wearing it, and each of the edges 31 of the absorber 30 may be covered.

On the other hand, in the first embodiment, at least a part of the gather 50 (the leakage preventing unit) is bonded with the front wing unit 60A at the front bonding portion 58A. In addition, the front bonding portion 58A is positioned in the vicinity of the reference line SL, specifically outside in the widthwise direction more than on the reference line SL. According to the embodiment, in a state in which the gather 50 is opened outside in the widthwise direction W of the absorbent article 1, the gather 50 is pulled to the outside in the widthwise direction W of the absorbent article 1, together with the front wing unit 60A. Thus, it is possible to prevent the gather 50 from falling down on the absorber 30 and to reliably restrain each of the edges 31 of the absorber 30 from being covered therewith. Therefore, it is possible to prevent a decrease of an absorption area of the absorber 30 due to falling down of the gather 50 and further reliably prevent a side leakage.

In the first embodiment, the gather 50 has elasticity in the longitudinal direction L of the absorbent article 1. According to the embodiment, the gather 50 reliably rises in the erection direction T (that is, the gather 50 reliably rises toward the wearer's side at the time of wearing it), thus making it possible to further reliably prevent a side leakage from the base part 61.

In the first embodiment, at least a part of the gather 50 (the leakage preventing unit) is bonded with the rear wing unit 60B at a rear bonding portion 158B. According to the embodiment, the base part 61 which is positioned between the front wing unit 60A and the rear wing unit 60B is reliably covered with the gather 50. Thus, a side leakage (such as an oblique leakage) from the base part 61 can be further reliably prevented.

In the first embodiment, the front bonding portion 58A is positioned at a position which includes the centerline CL1 as seen in the plan view of the absorbent article 1. In particular, it is preferable that the front bonding portion 58A is provided to be uniform with respect to the centerline CL1.

If the front bonding portion 58A is not provided to be uniform with respect to the centerline CL1, when the front wing unit 60A is folded back to a crotch portion of shorts, an elastic force of the cord-like member 80 that is positioned front or rear of the front bonding portion 58A is made different from usual.

In such a case, since the cord-like member 80 whose elastic force is strengthened is easily restored to a state before expansion, for example, only one-side front wing unit 60A may be easily restored to the side of the topsheet 10. Thus, it becomes cumbersome for a wearer to handle the absorbent article 1 when the article is worn on the shorts.

On the other hand, as described above, the front bonding portion 58A is provided to be uniform with respect to the centerline CL1, whereby the elastic force of the cord-line member 80 is maintained uniformly in the front wing unit 60A, thus facilitating handling of the absorbent article 1.

In the first embodiment, the front end portion 51A and the rear end portion 51B are bonded with the absorbent article 1 in a state in which the gather 50 is folded back a plurality of times with the fixed end 56 being a start point and then is folded on the topsheet 10. According to the embodiment, the gather 50 rises toward the wearer's side at the time of wearing it, and a hollow portion is formed in each of the inside leg region X1 and the rear region X2. Thus, the gather 50 is easily fitted to the wearer, and a side leakage from the base part 61 can be further reliably prevented.

In the first embodiment, the wing unit 60 is formed of a liquid impermeable material. In particular, it is preferable that the inside of the wing unit 60 or a face at the side of the topsheet 10 is formed of a liquid permeable material. According to the embodiment, even in a case where a bodily fluid passes through the top of the gather 50 and then a side leakage occurs, the bodily fluid can be absorbed by means of the wing unit 60. Thus, the side leakage from the base part 61 can be further reliably prevented.

In the first embodiment, it is preferable that the wing unit 60 is 150 mm or less in bending rigidity employing a cantilever. If the bending rigidity is less than 150 mm, the wing unit 60 is hardly taken along the shape of shorts, causing a wearer to have a sense of discomfort while in use.

In the first embodiment, the wing unit 60 is different from the gather 50 in hue. According to the embodiment, a wearer can easily distinguish the wing unit 60 and the gather 50 from each other, and even if the wing unit 60 is folded back to shorts, it is possible to visually indicate for the wearer's eyes the fact that the gather 50 is not folded back thereto.

In the first embodiment, it is preferable that the length Y1 in the widthwise direction W from the emboss part E to the bonding portion 58 is 1/2 or less, in particular 1/3 or less with respect to the length Y2 of the gather 50 from the emboss part E to the bonding portion 58. If the length Y1 is larger than 1/2 with respect to the length Y2, it may be that the gather 50 easily slackens, and the gather 50 hardly form a sufficient hollow portion, and a side leakage cannot be reliably prevented.

In the first embodiment, in the side part closed state of the absorbent article 1, from a boundary between the absorber 30 and the wing unit 60, the gather 50 is disposed on the topsheet 10, together with the wing unit 60, and the gather 50 is folded back in the erection direction T, a plurality of times, and then, is folded on the topsheet 10. According to the embodiment, in a case where the absorbent article 1 is packed, since the gather 50 and the wing unit 60 can be folded so as to be included in a width which is substantially identical to that of the absorber 30, the absorbent article can be compactly packed.

### [Second Embodiment]

Hereinafter, an absorbent article 1A according to a second embodiment according to the present invention will be described with reference to the drawings. The same constituent elements as those of the abovementioned absorbent article 1 according to the first embodiment are designated by the same reference numerals, and constituent elements which are different therefrom are mainly described.

Herein, in the abovementioned absorbent article 1 according to the first embodiment, the free end 57 is oriented to the outside in the widthwise direction W of the absorbent article 1 in a state in which the gather 50 is folded back in the erection direction T, a plurality of times, and is folded on the topsheet 10. On the other hand, in the absorbent article 1A according to the second embodiment, the free end 157 is oriented to the inside in the widthwise direction W of the absorbent article 1A in a state in which the gather 150 is folded back in the erection direction T at a plurality of times and then is folded on the topsheet 10.

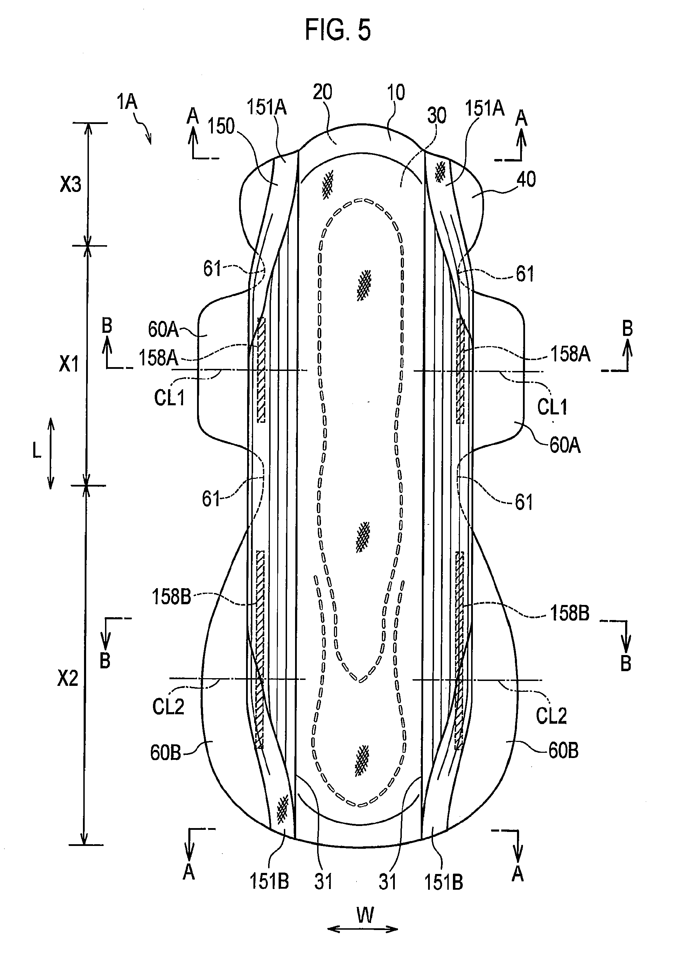

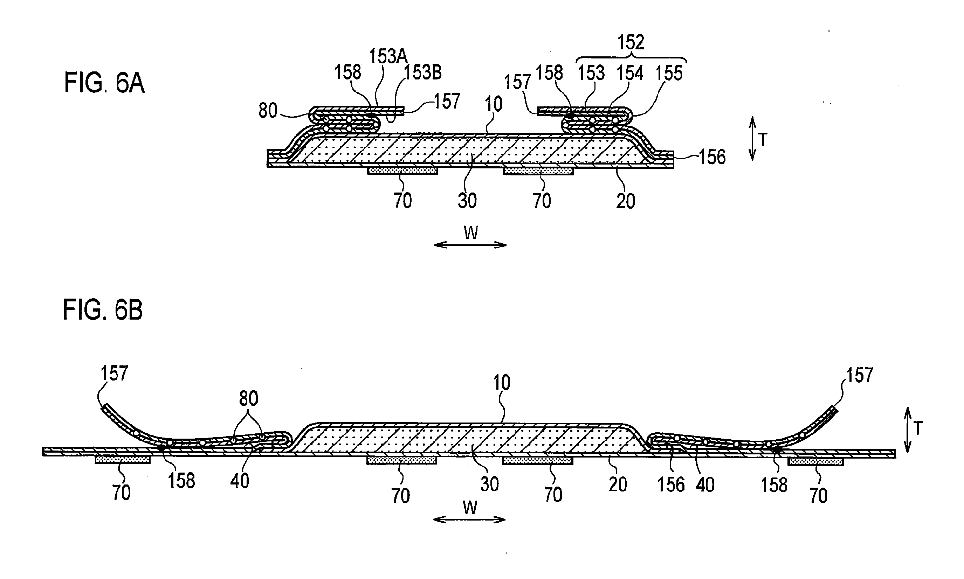

First, a structure of the gather 150 of the absorbent article 1A according to the second embodiment will be described with reference to Fig. 5 to Fig. 8. Fig. 5 is a plan view in a side part opened state of the absorbent article 1A according to the second embodiment. Fig. 6 (a) is a sectional view in the side part opened state of the absorbent article 1A according to the second embodiment (the sectional view taken along the line A-A of Fig. 5). Fig. 6 (b) is a sectional view in the side part opened state of the absorbent article 1A according to the second embodiment (the sectional view taken along the line B-B of Fig. 5).

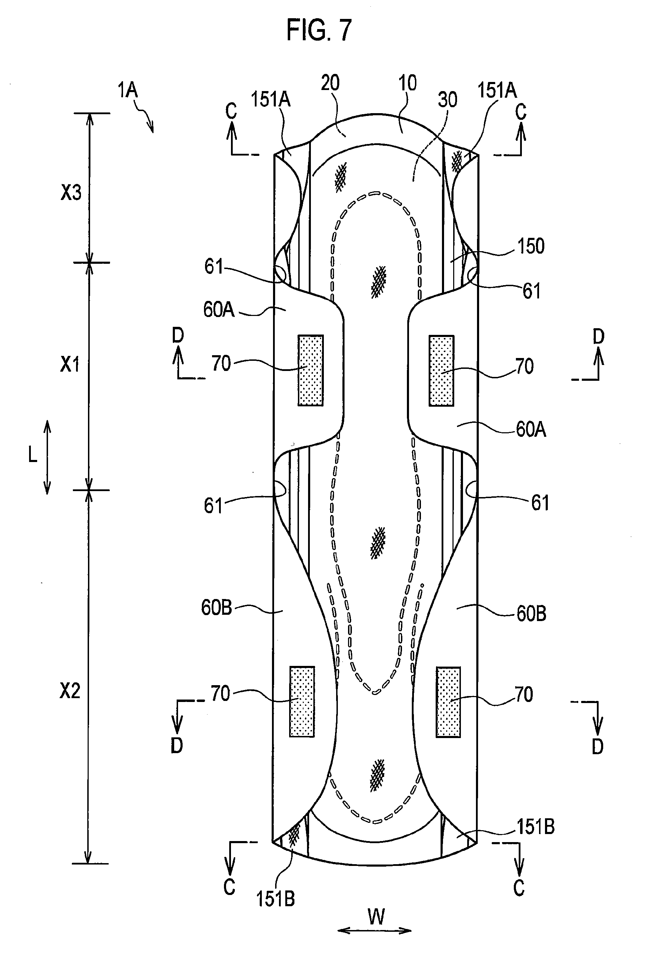

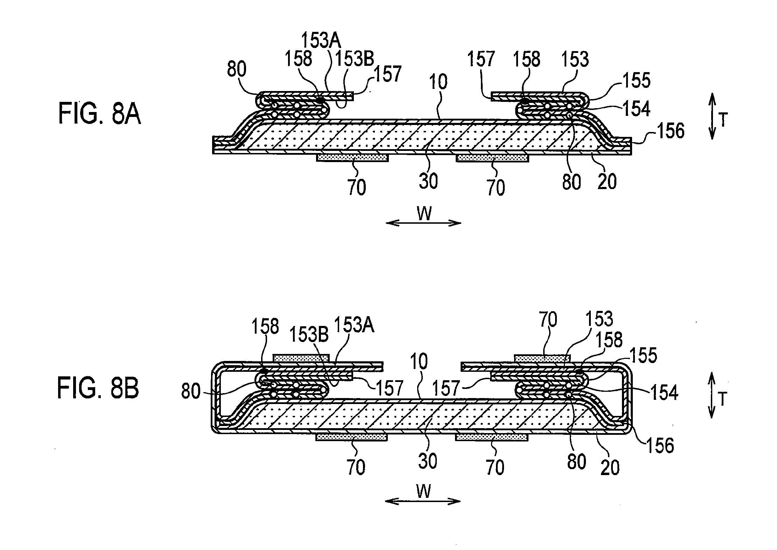

Fig. 7 is a plan view of a side part closed state of the absorbent article 1A according to the second embodiment. Fig. 8 (a) is a sectional view in the side part closed state of the absorbent article 1A according to the second embodiment (the sectional view taken along the line C-C of Fig. 7). Fig. 8 (b) is a sectional view in the side part closed state of the absorbent article 1A according to the second embodiment (the sectional view taken along the line D-D of Fig. 7).

As shown in Fig. 5 and Fig. 6, the gather 150 rises in the erection direction T, i.e., toward the wearer's side, at the time of wearing it. In addition, as shown in Fig. 7 and Fig. 8, the gather 150 is folded back a plurality of times onto the topsheet 10 in the side part closed state of the absorbent article 1A, that is, in a state in which the gather 150 does not rise.

The gather 150 is provided with: a front end portion 151A which is positioned at a front side of a wearer; and a rear end portion 151B which is positioned at a rear side of the wearer. The front end portion 151A and the rear end portion 151B are bonded with the absorbent article 1A in a state in which the gather 150 is folded on the topsheet 10. The gather 150 has a gather main unit 152, a fixed end 156, and a free end 157.

The gather main unit 152 is formed in a sheet shape which is provided along the longitudinal direction L of the absorbent article 1A. The gather main unit 152 has: at least an outermost piece portion 153 which is positioned at the most wearer's side in a state in which the gather 150 is folded back a plurality of times and is folded on the topsheet 10 as seen in a sectional view (refer to Fig. 8) in the widthwise direction W of the absorbent article 1A; a lower piece portion 154 which is positioned at the side of each of the edges 31 of the outermost piece portion 153; and a continuous portion 155 which connects the outermost piece portion 153 and the lower piece portion 154 to each other.

The outermost piece portion 153 has: an outermost face 153A which is positioned at the most wearer's side in the erection direction T of the gather 150; and an innermost face 153B which is positioned at an opposite side of the outermost face 153A, that is, at the side of the lower piece portion 154.

The fixed end 156 is provided along the longitudinal direction L of the absorbent article 1A, and is fixed to each of the edges 31. The fixed end 156 serves as a start point when the gather 50 rises in the erection direction T. Namely, the gather 150 rises in the erection direction T that is spaced from the topsheet 10 with the fixed end 156 being a start point.

The free end 157 is oriented to the inside in the widthwise direction W of the absorbent article 1A in a state in which the gather 150 is folded back in the erection direction T, a plurality of times and then is folded on the topsheet 10. Specifically, the free end 157 is positioned at a tip end part of the outermost piece portion 153, and is provided inside in the widthwise direction W of the absorbent article 1A more than the continuous portion 155.

Such a gather 150 is extensive to the outside in the widthwise direction W of the absorbent article 1A more than the base part 61 as seen in the plan view of the absorbent article 1A. The gather 150 is superimposed on the base part 61. In addition, at least a part of the gather 150 is bonded with the wing unit 60 at the bonding portion 158.

Next, a disposition site of the abovementioned bonding portion 158 will be described with reference to Fig. 5 to Fig. 8.

As shown in Fig. 5 to Fig. 8, the wing unit 60 and at least a part of the outermost piece portion 153 are bonded with each other at the bonding portion 158. Specifically, the side sheet 40 and the outermost face 153A of the outermost piece portion 153 are bonded with each other at the bonding portion 158 in a state in which the gather 150 is folded on the topsheet 10 a plurality of times.

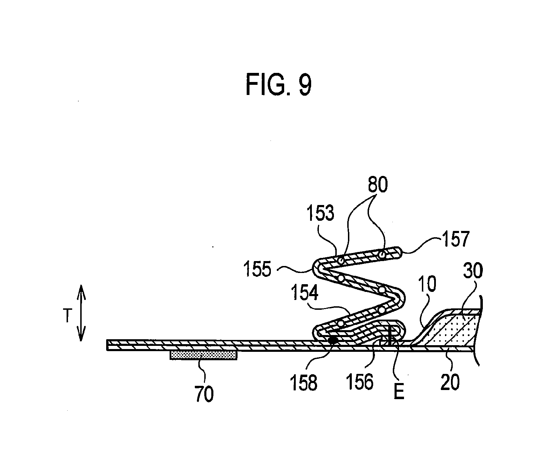

At the bonding portion 158, it is sufficient if the side sheet 40 and at least a part of the outermost face 153A are bonded with each other, and for example, as shown in Fig. 9, the side sheet 40 and a part of the lower piece portion 154 may be bonded with each other.

The bonding portion 158 is comprised of a front bonding portion 158A which is provided at a front wing unit 60A; and a rear bonding portion 158B which is provided at a rear wing unit 60B. The disposition sites of the front bonding portion 158A and the rear bonding portion 158B are similar to those of the front bonding portion 58A and the rear bonding portion 58B according to the first embodiment mentioned above.

With the absorbent article 1A according to the second embodiment, as is the case with the first embodiment, it is possible to reliably prevent a side leakage from the base part 61 and to disallow a wearer to have a sense of discomfort while in use. In addition, it becomes easy for the wearer to handle the absorbent article 1A when wearing the absorbent article on shorts.

### [Third Embodiment]

Hereinafter, an absorbent article 1B according to a third embodiment of the present invention will be described with reference to the drawings. The same constituent elements as those of the abovementioned absorbent article 1 according to the first embodiment are designated by the same reference numerals, and constituent elements which are different therefrom are mainly described.

Herein, in the abovementioned absorbent article 1 according to the first embodiment, the front end portion 51A and the rear end portion 51B of the gather 50 are bonded with the absorbent article 1 in a state in which the gather 50 is folded in the erection direction T, a plurality of times, and then, is folded on the topsheet 10. On the other hand, in the absorbent article 1B according to the third embodiment, a front end portion 251A and a rear end portion 251B of a gather 250 are bonded with the absorbent article 1B without being folded back on the topsheet 10 a plurality of times.

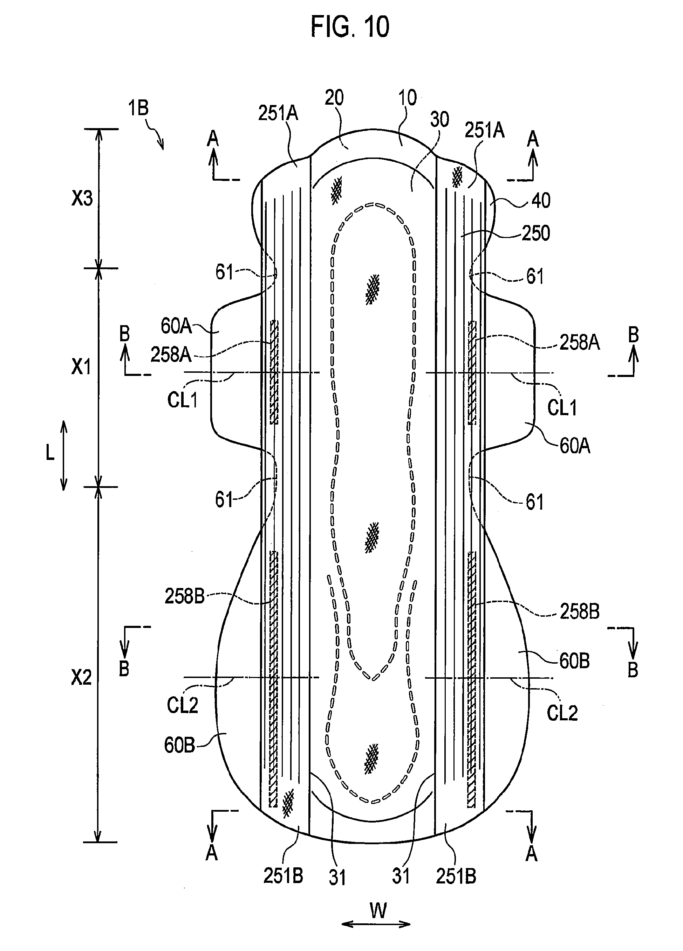

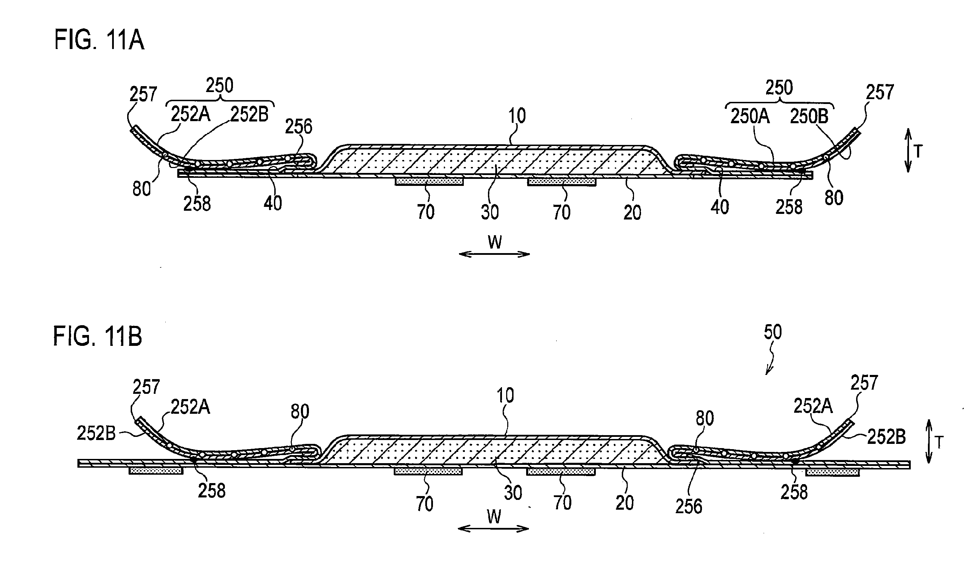

First, a structure of the gather 250 of the absorbent article 1B according to the third embodiment will be described with reference to Fig. 10 and Fig. 11. Fig. 10 is a plan view in a side part opened state of the absorbent article 1B according to the third embodiment. Fig. 11 (a) is a sectional view in the side part opened state of the absorbent article 1B according to the third embodiment (the sectional view taken along the line A-A of Fig. 10). Fig. 11 (b) is a sectional view in the side part opened state of the absorbent article 1B according to the third embodiment (the sectional view taken along the line B-B of Fig. 10). The third embodiment describes only the side part opened state of the absorbent article 1B.

As shown in Fig. 10 and Fig. 11, the gather 250 is provided along the longitudinal direction L of the absorbent article 1B from a front end portion 251A to a rear end portion 251B. Namely, the gather 250 is provided with: a frond end portion 251A which is positioned at a front side of a wearer; and a rear end portion 251B which is positioned at a rear side of the wearer. The front end portion 251A and the rear end portion 251B are bonded with the absorbent article 1B. The front end portion 251A and the rear end portion 251B are not folded on the topsheet 10. The gather 250 has a gather main unit 252, a fixed end 256, and a free end 257.

The gather main unit 252 is formed in a sheet shape which is provided along the longitudinal direction L of the absorbent article 1B. The gather main unit 252 has: an outside face 252A (an outermost face) which is positioned at the wearer's side as seen in a sectional view (refer to Fig. 11) in the widthwise direction W of the absorbent article 1B; and an inside face 252B which is positioned at an opposite side of the outermost piece portion 153, that is, at the side of the lower piece portion 154.

The fixed end 256 is provided along the longitudinal direction L of the absorbent article 1B, and is fixed to each of the edges 31. The fixed end 256 serves as a start point when the gather 250 rises in the erection direction T. Namely, the gather 250 rises in the erection direction T which is spaced from the topsheet 10 with the fixed end 256 being a start point.

The free end 257 is oriented to the outside in the widthwise direction W of the absorbent article 1B in a state in which the gather 250 is folded in the erection direction T once and then is folded on the topsheet 10.

Such a gather 250 is extensive to the outside in the widthwise direction W of the absorbent article 1B more than the base part 61 as seen in a plan view of the absorbent article 1B. Namely, the gather 250 is superimposed on the base part 61. In addition, at least a part of the gather 250 is bonded with the wing unit 60 at the bonding portion 258.

Next, a disposition site of the abovementioned bonding portion 258 will be described with reference to Fig. 10 and Fig. 11.

As shown in Fig. 10 and Fig. 11, the wing unit 60 and at least a part of the inside face 252B, are bonded with each other at the bonding portion 258. Specifically, the side sheet 40 and an at least a part of the inside face 252B are bonded with each other at the bonding portion 258.

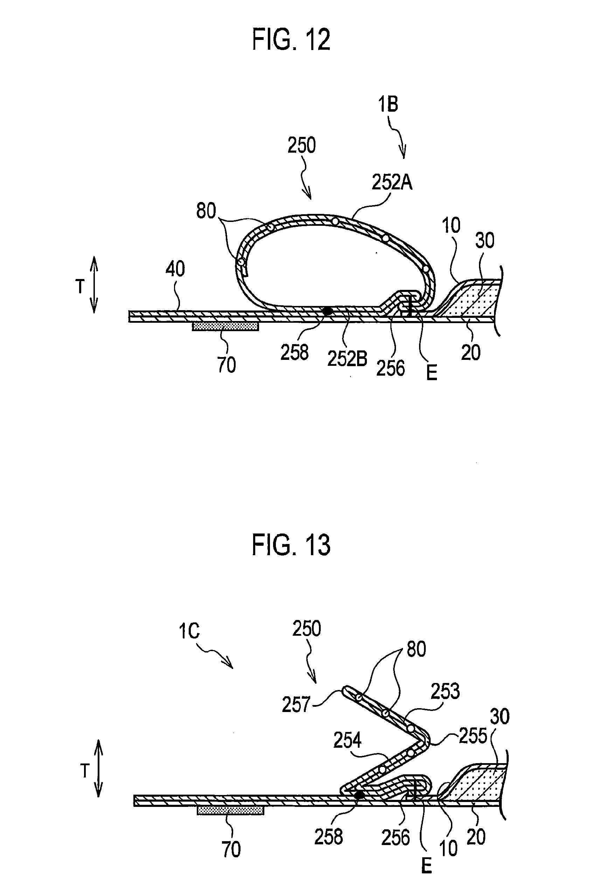

At the bonding portion 258, it is sufficient if the side sheet 40 and a part of the inside face 252B are bonded with each other, and for example, as shown in Fig. 12, the side sheet 40 and the inside face 252B of the gather 50 forming a hollow portion may be bonded with each other.

The bonding portion 258 is comprised of a front bonding portion 258A which is provided at a front wing unit 60A; and a rear bonding portion 258A which is provided at a rear wing unit 60B. Disposition sites of the front bonding portion 258A and the rear bonding portion 258B are similar to those of the front bonding portion 58A and the rear bonding portion 58B according to the first embodiment mentioned above.

Next, an exemplary modification of the absorbent article 1B according to the third embodiment will be described with reference to the drawings. The same constituent elements as those of the abovementioned absorbent article 1B according to the third embodiment are designated by the same reference numerals, and constituent elements which are different therefrom are mainly described.

First, the exemplary modification of the absorbent article 1B according to the third embodiment will be described with reference to Fig. 13. Fig. 13 is a sectional view in a side part opened state of an absorbent article 1C according to Exemplary Modification 1.

Herein, in the abovementioned absorbent article 1B according to the third embodiment, a gather main unit 252 has an outside face 252A and an inside face 252B. On the other hand, in the absorbent article 1C according to Exemplary Modification 1, a gather main unit 252 has an outermost piece portion 253, a lower piece portion 254, and a continuous portion 255.

A wing unit 60 and at least a part of the lower piece portion 254 are bonded with each other at a bonding portion 258. Specifically, a side sheet 40 and a part of the lower piece portion 254 are bonded with each other at the bonding portion 258.

The free end 257, as shown in Fig. 13, may be oriented to the outside in a widthwise direction W of the absorbent article 1C or may be oriented to the inside in the widthwise direction W of the absorbent article 1C.

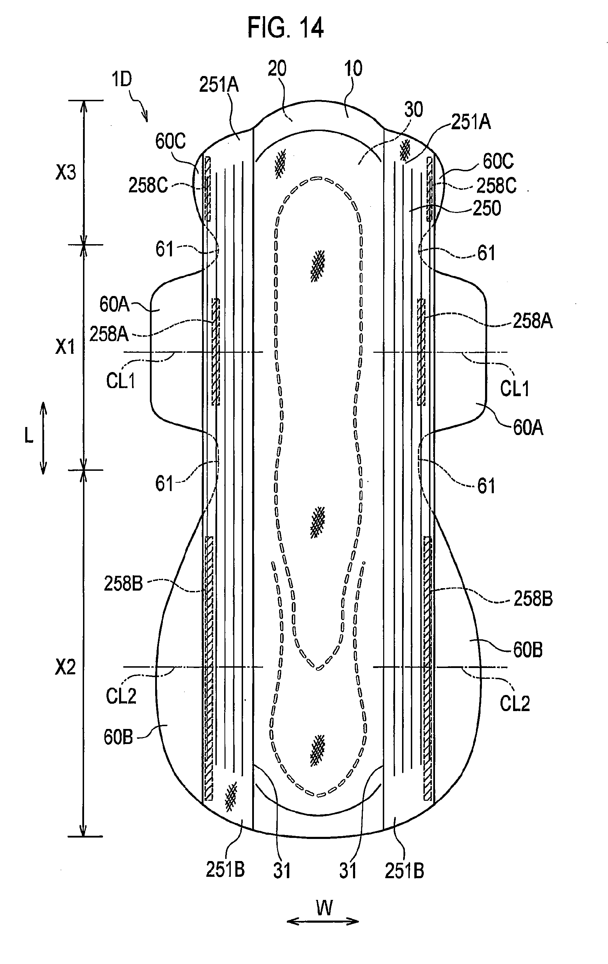

First, an exemplary modification of the absorbent article 1B according to the third embodiment will be described with reference to Fig. 14. Fig. 14 is a plan view in a side part opened state of an absorbent article 1D according to Exemplary Modification 2.

Herein, in the abovementioned absorbent article 1B according to the third embodiment, a bonding portion 258 is comprised of a front bonding portion 258A and a rear bonding portion 258B. On the other hand, in the absorbent article 1D according to Exemplary Modification 2, a bonding portion 258 is comprised of a front bonding portion 258A, a rear bonding portion 258B, and a front end bonding portion 258C. The front bonding portion 258A and the rear bonding portion 258B are similar to the front bonding portion 58A and the rear bonding portion 58B that were described in the first embodiment.

The front end bonding portion 258C is provided at a front end wing unit 60C which is extensive to the outside in the widthwise direction W of the absorber 30 in a front region X3 which corresponds to a front side of a wearer more than the front wing unit 60A. It is preferable that the front end bonding portion 258C is positioned in the vicinity of the reference line SL as is the case with the front bonding portion 58A and the rear bonding portion 58B that were described in the first embodiment.

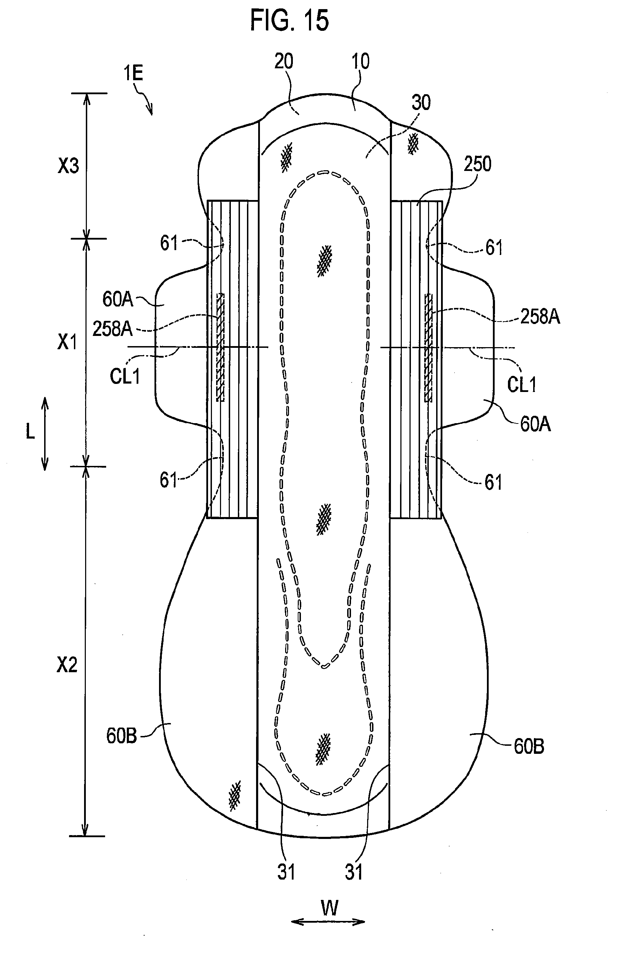

Next, an exemplary modification of the absorbent article 1B according to the third embodiment will be described with reference to Fig. 15. Fig. 15 is a plan view in a side part opened state of an absorbent article 1E according to Exemplary Modification 3.

Herein, in the abovementioned absorbent article 1B according to the third embodiment, a gather 250 is provided along a longitudinal direction L of the absorbent article 1B from a front end portion 251A to a rear end portion 251B. On the other hand, in the absorbent article 1E according to Exemplary Modification 3, a gather 250 is provided only in the vicinity of a front wing unit 60A.

Specifically, in the absorbent article 1, the gather 250 is provided at each of the edges 31 which is positioned outside in the widthwise direction W of the absorber 30, and is extensive along the longitudinal direction L of the absorbent article 1. The gather 250 is provided from a base part 61 which is positioned between a front wing unit 60A and a front end wing unit 60C to a base part 61 which is positioned between a rear wing unit 60B and the front end wing unit 60C.

With the absorbent articles 1B to 1E according to the third embodiment, as is the case with the first embodiment and the second embodiment, the absorbent article 1B can be easily handled without causing a wearer to have a sense of discomfort while in use, and a side leakage from the base part 61 can be further reliably prevented.

### [Fourth Embodiment]

Hereinafter, an absorbent article 1F according to a fourth embodiment of the present invention will be described with reference to the drawings. The same constituent elements as those of the abovementioned absorbent article 1 according to the first embodiment are designated by the same reference numerals, and portions which are different therefrom are mainly described.

Herein, in the abovementioned absorbent article 1 according to the first embodiment, at least part of the gather 50 has a bonding portion 58. On the other hand, in the absorbent article 1F according to the fourth embodiment, a gather 350 does not have a bonding portion.

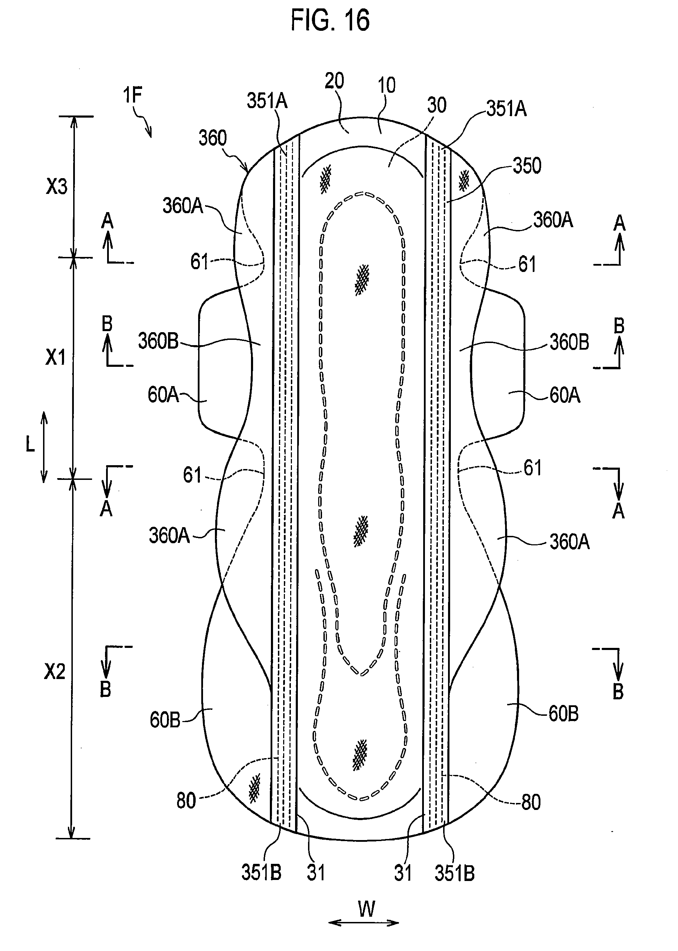

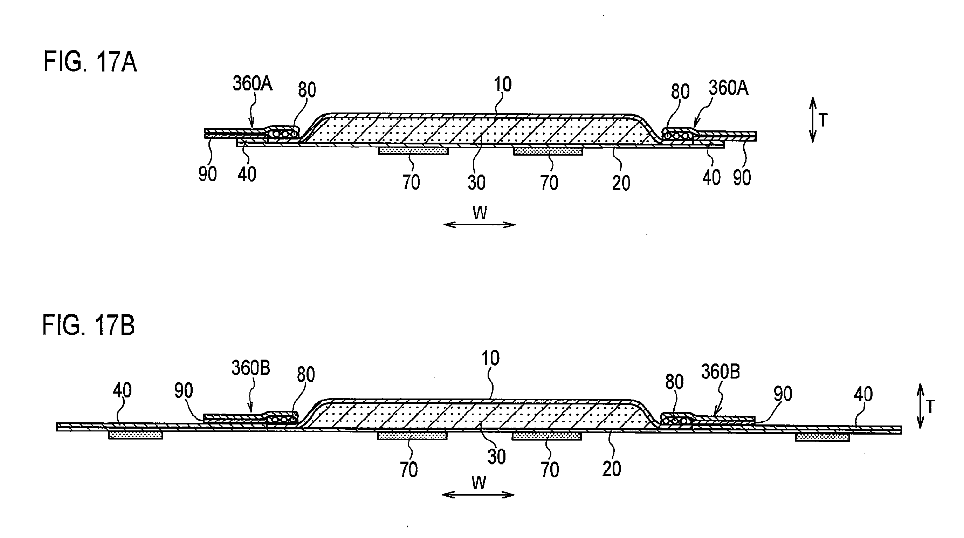

First, a structure of the gather 350 of the absorbent article 1F according to the fourth embodiment will be described with reference to Fig. 16 and Fig. 17. Fig. 16 is a plan view in a side part opened state of the absorbent article 1F according to the fourth embodiment. Fig. 17 (a) is a sectional view in the side part opened state of the absorbent article 1F according to the fourth embodiment (the sectional view taken along the line A-A of Fig. 16). Fig. 17 (b) is a sectional view in the side part opened state of the absorbent article 1F according to the fourth embodiment (the sectional view taken along the line B-B of Fig. 16). The fourth embodiment describes only the side part opened state of the absorbent article 1F.

As shown in Fig. 16 and Fig. 17, the absorbent article 1F has a cover part 360 (a leakage preventing unit) in addition to the gather 350. Inside of the gather 350, a cord-like member 80 is provided in an expanded state. The gather 350 is provided with: a front end portion 351A which is positioned at a front side of a wearer; and a rear end portion 351B which is positioned at a rear side of the wearer. The front end portion 351A and the rear end portion 351B are folded back onto a topsheet 10 a plurality of times.

The cover part 360 is extensive to the outside in a widthwise direction W of the absorbent article 1F. The cover part 360 is provided at the side of the topsheet 10 more than a wing unit 60 (a front wing unit 60A and a rear wing unit 60B).

The cover 360 is composed of the gather 350; and a water resistance sheet 90 which is positioned at the side of a backsheet 20. It is a matter of course that a water repellent or hydrophobic unwoven cloth or the like may be employed in place of the water resistance sheet 90.

The cover part 360 has: a long extension portion 360A which is long in length which is extensive from an absorber 30 to the outside in a widthwise direction; and a short extension portion 360B which is short in length which is extensive from the absorber 30 to the outside in the widthwise direction.

The long extension portion 360A is positioned on a base part 61 as seen in a plan view of the absorbent article 1F. The long extension portion 360A is extensive to the outside in the widthwise direction W of the absorbent article 1F more than the base part 61 as seen in the plan view of the absorbent article 1F. Namely, the long extension portion 360A is superimposed on the base part 61.

The short extension portion 360B is positioned on a wing unit 60 (a front wing unit 60A and a rear wing unit 60B) as seen in the plan view of the absorbent article 1F. The short extension portion 360B is short in length which is extensive from the absorber 30 to the outside in the widthwise direction more than the wing unit 60.

Herein, while, in the fourth embodiment, the cover part 360 is not bonded with the wing unit 60 (the front wing unit 60A or the rear wing unit 60B and the front end wing unit 60C), the cover part 360 may be bonded with the wing unit 60. In addition, the absorbent article 1F does not always need to have the gather 350, and it is sufficient if the absorbent article has the cover part 360.

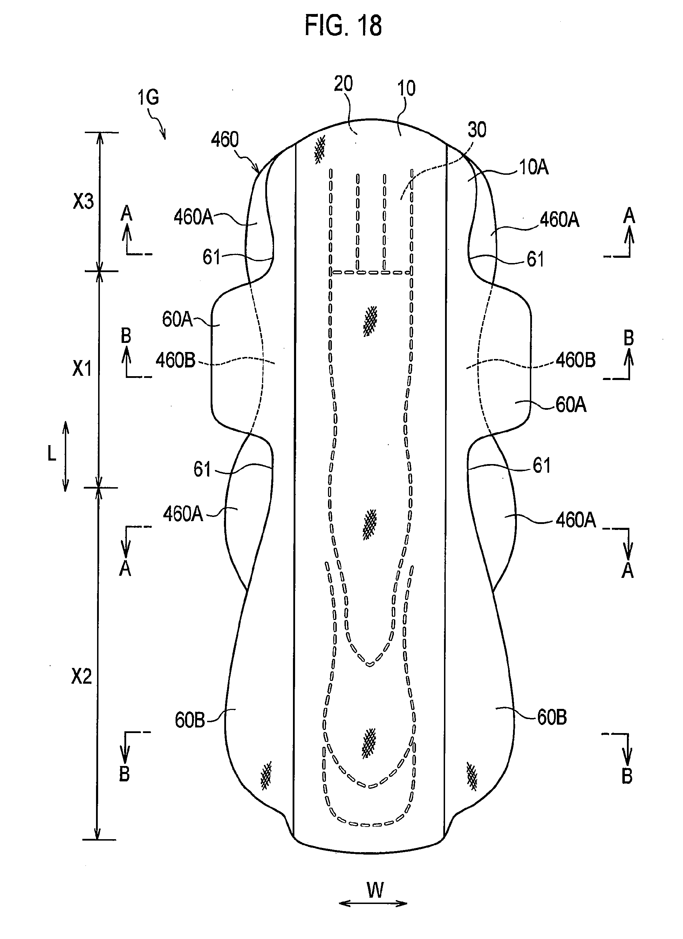

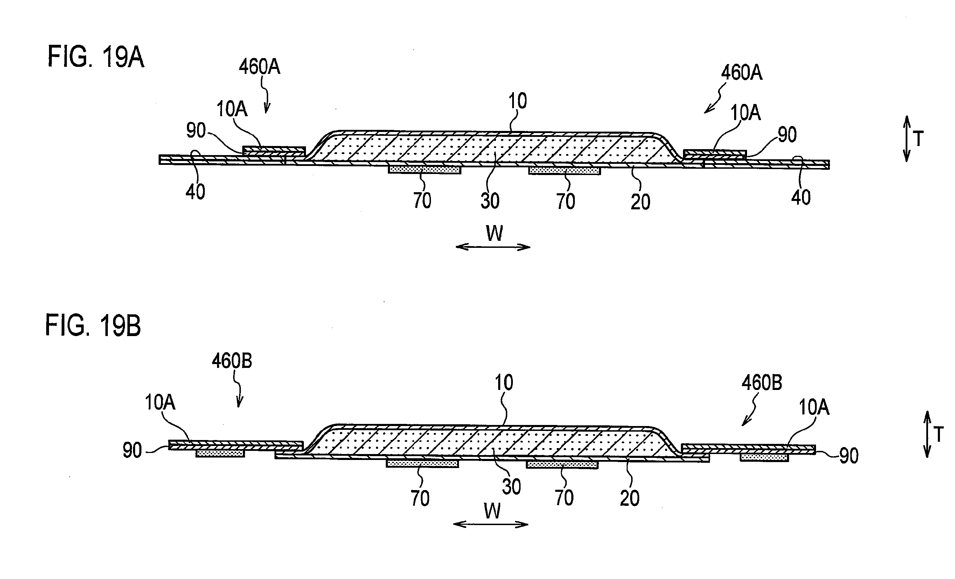

Next, an exemplary modification of the absorbent article 1F according to the fourth embodiment will be described with reference to Fig. 18 and Fig. 19. Fig. 18 is a plan view in a side part opened state of an absorbent article 1G according to the exemplary modification. Fig. 19 (a) is a sectional view in the side part opened state of the absorbent article 1G according to the exemplary modification (the sectional view taken along the line A-A of Fig. 18). Fig. 19 (b) is a sectional view in the side part opened state of the absorbent article 1G according to the exemplary modification (the sectional view taken along the line B-B of Fig. 18).

Herein, in the abovementioned absorbent article 1F according to the fourth embodiment, a cover part 360 is provided at the side of a topsheet 10 more than a wing unit 60. On the other hand, in the absorbent article 1G according to the exemplary modification, a cover part 460 is provided at the side of a backsheet 20 more than the wing unit 60.

Specifically, as shown in Fig. 18 and Fig. 19, the absorbent article 1G has the cover part 460 which is superimposed on the wing unit 60. The cover part 460 is comprised of: a topside sheet 10A made of the same member as the topsheet 10; and a water resistance sheet 90 which is positioned at the side of the backsheet 20. The cover part 460 has a long extension portion 460A which is long in length which is extensive from the absorber 30 to the outside in a widthwise direction; and a short extension portion 460B which is short in length which is extensive from the absorber 30 to the outside in the widthwise direction.

The long extension portion 460A is positioned on a base part 61 as seen in a plan view of the absorbent article 1G. The long extension portion 460A is extensive to the outside in a widthwise direction W of the absorbent article 1G more than the base part 61 as seen in the plan view of the absorbent article 1G. Namely, the long extension portion 460Ais superimposed on the base part 61.

The short extension portion 460B is positioned on the wing unit 60 (the front wing unit 60A and the rear wing unit 60B) as seen in the plan view of the absorbent article 1G. The short extension portion 460B is short in length which is extensive from the absorber 30 to the outside in a widthwise direction more than the wing unit 60.

According to the absorbent articles 1F, 1G according to the fourth embodiment, as is the case with the first embodiment, the second embodiment, and the third embodiment, the absorbent articles 1F, 1G can be easily handled without causing a wearer to have a sense of discomfort while in use, and a side leakage from the base part 61 can be further reliably prevented.

### [Other Embodiments]

As described above, while the contents of the present invention were disclosed through the embodiments of the present invention, it should not be understood that the discussions and drawings forming a part of this disclosure limit the present invention. From this disclosure, a variety of alternative aspects, examples, and operational techniques would be self-evident to one skilled in the art.

For example, the embodiments of the present invention can be modified as follows. Specifically, while the embodiments described that an absorbent article 1 is employed as a sanitary napkin, the absorbent article may be an incontinent pad or a panty liner, a disposable diaper or the like, for example, without being limitative thereto.

In addition, while the embodiments described that a wing unit 60 is comprised of a backsheet 20 and a side sheet 40, it is a matter of course that: the wing unit may be comprised of the topsheet 10 and the backsheet 20, for example, without being limitative thereto; and the wing unit 60 may be formed of a sheet other than the topsheet 10 or the backsheet 20.

Further, while the embodiments described that the wing unit 60 is comprised of a front wing unit 60A and a rear wing unit 60B, the wing unit 60 may be a portion which is extensive to the outside in the widthwise direction W more than an absorber 30 without being limitative thereto.

Furthermore, while the embodiments described that a displacement proof unit 70 is provided on a face at the side of the backsheet 20 in the wing unit 60, it is a matter of course that the displacement proof unit 70 may not be provided without being limitative thereto.

Still furthermore, while the embodiments described that the wing unit 60 is different from a gather 50 in hue, the wing unit 60 may be identical to the gather 50 in hue without being limitative thereto.

Yet furthermore, while the embodiments described that, inside of the gather 50, the cord-like member 80 is provided in an expanded state, the cord-like member 80 may not be provided without being limitative thereto. For example, a sheet per se constituting the gather 50 may have expandability. It is a matter of course that the shape or folding back of the gather 50 may be different from the one described in the foregoing embodiments.

In addition, while the embodiments described that at least a part of the gather 50 has a bonding portion 58, it may not have the bonding portion 58 without being limitative thereto.

Further, while the embodiments described that the bonding portion 58 is bonded with the wing unit 60 by means of an adhesive agent such as a hot melt adhesive, for example, the bonding portion 58 may be bonded with the wing unit 60 by means of emboss or sonic sealing or the like.

Furthermore, while the embodiments described that a bonding portion 58 (a front bonding portion 58A and a rear bonding portion 58B) is positioned in the vicinity of a reference line SL, for example, the bonding portion 58 may be positioned on the reference line SL or may be positioned inside or outside in a widthwise direction W of the absorbent article 1 with respect to the reference line SL, without being limitative thereto.

Still furthermore, while the embodiments described that a length L1 taken along a longitudinal direction L of the absorbent article 1 at the front bonding portion 58A is larger than a length L2 taken along the longitudinal direction L of the absorbent article 1 at the displacement proof unit 70, the length L1 may be identical to or shorter than the length L2 without being limitative thereto.

Yet furthermore, while the embodiments described that the front bonding portion 58A is provided at a position which includes a centerline CL1 as seen in a plan view of the absorbent article 1, this bonding portion 58 may be provided at a position which does not include the centerline CL1 (for example, forward or rearward of the centerline CL1) without being limitative thereto.

Moreover, while the embodiments described that the rear bonding portion 58B is provided at a position which includes a centerline CL2 as seen in a plan view of the absorbent article 1, this bonding portion 58 may be provided at a position which does not include the centerline CL2 (for example, forward or rearward of the centerline CL2) without being limitative thereto.

As described above, it is a matter of course that the present invention encompasses a variety of embodiments which have not been described herein. Therefore, technical scope of the present invention is defined only by the specific matters of the invention according to the claims that are reasonable from the foregoing description.

The entire contents of Japanese Patent Application No. 2009-002086 (filed on January 7, 2009) are incorporated in the present specification by way of reference.

### [Industrial Applicability]

As described above, according to the present invention, a side leakage can be reliably prevented, since a leakage preventing unit is extensive to the outside in a widthwise direction of an absorbent article more than a base part of a wing unit. In addition, even if the wing unit is folded back to shorts, the base part of the wing unit is never folded back, together with the wing unit, and thus, wrinkles do not occur at a topsheet or a front wing unit, and a wearer does not have a sense of discomfort while in use. Therefore, the present invention is effective for use in an absorbent article such as a sanitary napkin, an incontinent pad, or a panty liner.

## Claims

1. An absorbent article having: a liquid permeable topsheet; a liquid impermeable backsheet; and an absorber which is provided between the topsheet and the backsheet, said absorbent article comprising:
a front wing unit which is provided outside in a widthwise direction of the absorbent article more than the absorber, and is extensive to an outside in a widthwise direction of the absorber in an inside leg region which corresponds to an inside leg portion of a wearer; and
a leakage preventing unit which is provided at each of edges of the absorber, which is extensive along a longitudinal direction of the absorbent article, and which prevents a bodily fluid of the wearer from leaking from the absorbent article, wherein:
the front wing unit has a base part which is positioned at an innermost side in a widthwise direction of the absorbent article in the front wing unit; and
the leakage preventing unit is extensive to an outside in the widthwise direction of the absorbent article more than the base part as seen in a plan view of the absorbent article.

2. The absorbent article according to claim 1, wherein: at least a part of the leakage preventing unit has a front bonding portion which is to be bonded with the front wing unit; and the front bonding portion is positioned on a reference line which passes through the innermost side in the widthwise direction of the base part, and which is extensive along a longitudinal direction of the absorbent article or outside in a widthwise direction more than on the reference line.

3. The absorbent article according to claim 1 or 2, further comprising a rear wing unit which is provided outside in the widthwise direction of the absorbent article more than the absorber, and which is extensive to the outside in the widthwise direction of the absorber in a rear region which corresponds to a rear side of the wearer more than the front wing unit,
wherein at least a part of the leakage preventing unit has a rear bonding portion to be bonded with the rear wing unit.

4. The absorbent article according to any one of claims 1 to 3, wherein the leakage preventing unit has elasticity in the longitudinal direction of the absorbent article.

5. The absorbent article according to any one of claims 2 to 4, wherein the front bonding portion is provided at a position which includes a centerline which passes through a center in the longitudinal direction of the absorbent article in the front wing unit and which is extensive along the widthwise direction of the absorbent article.

6. The absorbent article according to any one of claims 1 to 5, wherein:
the leakage preventing unit comprises:
a fixed end which is fixed at each of the edges;
a front end portion which is positioned at a front side of the wearer; and
a rear end portion which is positioned at a rear side of the wearer; and
the front end portion and the rear end portion are bonded with the absorbent article in a state in which the front and rear end portions are folded back in an erection direction which is spaced from the topsheet with the fixed end being a start point and then are folded on the topsheet.

7. The absorbent article according to claim 6, wherein:
the leakage preventing unit comprises a free end which is provided in the longitudinal direction of the absorbent article, and which serves as the other end of the fixed ends;
the free end is oriented to the outside in the widthwise direction of the absorbent article in a state in which the leakage preventing unit is folded back in the erection direction and then is folded on the topsheet.

8. The absorbent article according to claim 6, wherein:
the leakage preventing unit has a free end which is provided along the longitudinal direction of the absorbent article, and which serves as the other end of the fixed ends; and
the free end is oriented to an inside in the widthwise direction of the absorbent article in a state in which the leakage preventing unit is folded back in the erection direction and then is folded on the topsheet.

9. The absorbent article according to claim 7 or 8, wherein:
the leakage preventing unit has an outermost face which is positioned at the most wearer's side in a state in which the leakage preventing unit is folded back in the erection direction and then is folded on the topsheet; and
at least a part of the outermost face is bonded with the front wing unit at the front bonding portion.

10. The absorbent article according to claim 9, further comprising:
a rear wing unit which is provided outside in the widthwise direction of the absorbent article more than the absorber, and which is extensive to the outside in the widthwise direction of the absorber in a rear region which corresponds to the rear side of the wearer more than the front wing unit,
wherein at least a part of the outermost face has a rear bonding portion which is bonded with the rear wing unit.
